# EUROPEAN PATENT APPLICATION

(11) **EP 4 574 990 A1**
(43) Date of publication of application: **25.06.2025**
(21) Application number: 23220136.8
(22) Date of filing: 22.12.2023
(51) Int. Cl.: C12P 19/34, C12M 1/00, C12P 21/02

(54) **METHODS AND SYSTEMS FOR PRODUCING PEPTIDES AND PROTEINS**

(71) Applicant: Nederlandse Organisatie voor toegepast-natuurwetenschappelijk Onderzoek TNO, 2595 DA 's-Gravenhage (NL)
(72) Inventor: ÁLVAREZ BERMÚDEZ, Olaia, 2595 DA s'-Gravenhage (NL); PELAY GIMENO, Marta, 2595 DA s'-Gravenhage (NL); VAES, Wouter Henricus Johannes, 2595 DA s'-Gravenhage (NL); VAN DUIJN, Petra Wilhelmina, 2595 DA s'-Gravenhage (NL); VERSLUIS, Michelle Maria, 2595 DA s'-Gravenhage (NL); DE MATTOS, Eduardo, 2595 DA s'-Gravenhage (NL)
(74) Representative: V.O.

(57) **Abstract**

The invention relates to a method for *in vitro* synthesis of a peptide or protein comprising transcribing a DNA into RNA in a first reaction container comprising the DNA and an enzyme capable of transcribing the DNA into RNA, introducing the RNA into a second reaction container, and translating the RNA into a peptide or protein in the second reaction container, wherein the first reaction container and the second reaction container are connected. The invention further relates to a system for reconstituted cell-free protein synthesis useful in such method and uses thereof.

## Description

### Field of the invention

The invention relates to the field of peptide and protein synthesis. More specifically, the invention relates to reconstituted cell-free protein synthesis and systems for use therein.

### Background of the invention

Biologicals are increasingly developed for a wide variety of therapeutic applications, traditionally dominated by small molecule drugs. Large biomolecules (from peptides to antibodies and other therapeutic proteins) represent >50% of the new drugs approved by regulatory agencies.

Currently, these therapeutic peptides and proteins are mainly produced by expression technologies, like recombinant expression, using living cells. When used for therapeutic applications, these biological production systems are characterized by intrinsic issues such as heterogeneous protein production with batch-to-batch variations, low efficiency amongst others due to the primary objective of cells of staying alive, large amounts of waste material and need for (batch-wise) expensive biological safety testing. Consequently, production batches fail regularly, resulting in high production costs. As a result, costs of goods for therapeutic proteins are >100 times higher than for chemically synthesized small molecule alternatives.

Chemical synthesis alternatives, like solid-phase peptide synthesis or SPPS, are still being used for industrial production of homogeneous batches of small peptides, especially when they contain special features in their structure. However, industry is nowadays looking for more sustainable approaches, also suitable for synthesis of larger biomolecules.

In this context, alternative cell-free protein synthesis technologies that mimic the cellular processes are being developed and are attracting a great interest. They provide special added value for the production of hard to express proteins, often due to toxicity or folding issues. Moreover, cell-free strategies bring a new potential to solve intrinsic problems from cell expression, e.g., recombinant expression, while accelerating discovery timelines and simplifying purification requirements for therapeutic applications. Furthermore, they offer sustainability advantages for incorporating non-natural amino acids or achieving specific post-translation modifications (e.g., in the middle of sequence or C-terminal amides), typically attainable through chemical means. Most of the available cell-free platforms are based on the use of cell extracts, e.g., from *Escherichia coli,* some of which, have scale-up capabilities for large scale production. Lysate-based systems are obtained by disrupting the cell membrane of a cell, thereby extracting the intracellular ingredients. Subsequently, the non-soluble parts of the lysed cell can be removed, after which the lysate may be subjected to one or more purification steps. A disadvantage of lysate-based systems is the presence of intracellular ingredients that may not be involved in protein synthesis and may affect the *in vitro* processes in unexpected and/or negative ways. Hence, these systems come with intrinsic challenges and limitations in terms of control of the reaction composition, yield optimization and/or purification requirements. A second approach uses a reconstituted minimal enzymatic system enabling reaction composition control. This strategy is based on one-pot reactions, wherein DNA transcription and mRNA translation are performed in a single reaction container. Reconstituted systems are systems where the individual components or components' classes, such as enzymes, ribosomes and tRNAs, of the transcription and translation machinery are purified and added along with the DNA template. An advantage of such systems is that they are largely free of cell components that have no function supporting, or even interfere with, transcription and translation.

WO 2002/053582 and Shimizu et al. (2001) describe the so-called PURE system, a reconstituted cell-free protein synthesis system using recombinant enzymes for synthesizing peptides and proteins *in vitro.* Enzymes constituting the transcription/translation reaction system are labelled, which allows for isolation of the synthesized protein using affinity chromatography to remove the tagged enzymes.

Such reconstituted cell-free protein synthesis methods makes use of expensive enzymatic machinery, thus becoming unaffordable for large-scale purposes. Additionally, it is difficult to control and optimize the reaction conditions for the constituting enzymatic reactions, like transcription and translation.

Accordingly, there is a need in the art for further development of new cell-free protein synthesis technologies.

### Summary of the invention

It is an object of the present invention to provide methods and systems to produce peptides and proteins in a cell-free system that overcome one or more of the disadvantages described above.
a) The invention therefore provides a method for *in vitro* synthesis of a peptide transcribing a DNA into RNA in a first reaction container comprising the DNA and an enzyme capable of transcribing DNA into RNA,
b) introducing the RNA into a second reaction container, and
c) translating the RNA into a peptide or protein in the second reaction container,
wherein the first reaction container and the second reaction container are connected.

In a further aspect, the invention provides a system for reconstituted cell-free protein synthesis, comprising:
- a first reaction container comprising a reaction solution comprising an enzyme capable of transcribing DNA into RNA and ribonucleotides, preferably nuceloside triphosphates (NTPs),
- a second reaction container comprising a reaction solution comprising ribosomes, tRNAs, amino acids, aminoacylated tRNAs, catalysts for tRNAs aminoacylation and/or translation factors for protein synthesis, and
- a conduit for conducting RNA from the first reaction container into the second reaction container.

In a further aspect, the invention provides a use of a system of the invention for the synthesis of a peptide or protein.

In preferred embodiments, a method of the invention comprises the use of a system according to the invention.

In preferred embodiments, the method is performed using a reconstituted cell-free protein synthesis system.

In preferred embodiments, the components constituting the reconstituted cell-free protein synthesis system are purified components.

In preferred embodiments, the first reaction container comprises DNA immobilized on a solid support.

In preferred embodiments, the first reaction container further comprises a reaction solution containing a buffer and ribonucleotides, preferably nucleoside triphosphates (NTPs).

In preferred embodiments, the second reaction container comprises ribosomes, tRNAs, amino acids, aminoacylated tRNAs, catalysts for tRNA aminoacylation and/or translation factors for protein synthesis.

In preferred embodiments, the first reaction container comprises an access for insertion of a solid support comprising DNA immobilized thereto into the first reaction container such that the immobilized DNA is in contact with the reaction solution comprising the enzyme capable of transcribing DNA into RNA and the ribonucleotides.

In preferred embodiments, the system further comprises a separation module for separating the enzyme capable of transcribing DNA into RNA and RNA, which interrupts the conduit for conducting RNA from the first reaction container to the second reaction container.

In preferred embodiments, the method further comprises separating the DNA and the RNA prior to introducing RNA into the second reaction container.

In preferred embodiments, the method further comprises separating the enzyme capable of transcribing DNA into RNA and the RNA prior to introducing the RNA into the second reaction container.

In preferred embodiments, separating of the enzyme capable of transcribing DNA into RNA and the RNA is performed in a separation module.

In preferred embodiments, the enzyme capable of transcribing DNA into RNA that is separated from the RNA is reintroduced into the first reaction container.

In preferred embodiments, the method is performed in a continuous or semi-continuous manner, whereby the RNA is continuously or semi-continuously introduced into the second reaction container and/or the enzyme capable of transcribing DNA into RNA that is separated from the RNA is continuously or semi-continuously reintroduced into the first reaction container.

In preferred embodiments, a method of the invention comprises the use of a system according to the invention.

### Detailed description

As used herein, "to comprise" and its conjugations is used in its non-limiting sense to mean that items following the word are included, but items not specifically mentioned are not excluded. In addition, the verb "to consist" may be replaced by "to consist essentially of" meaning that a compound or adjunct compound as defined herein may comprise additional component(s) than the ones specifically identified, said additional component(s) not altering the unique characteristic of the invention.

The articles "a" and "an" are used herein to refer to one or to more than one (i.e., to at least one) of the grammatical object of the article. By way of example, "an element" means one element or more than one element.

The word "approximately" or "about" when used in association with a numerical value (e.g., approximately 10, about 10) preferably means that the value may be the given value (e.g., 10), plus or minus 5% of the value (e.g., 10, plus or minus 5%), preferably plus or minus 1% of the value.

The use of the alternative (e.g., "or") should be understood to mean either one, both, or any combination of the alternatives.

The present inventors have developed a novel platform technology for cell-free peptide and protein synthesis. The technology is based on the deconvolution of cellular protein expression, allowing to obtain a minimal enzymatic system containing the essential machinery needed for protein synthesis. The main biochemical reactions of the process, transcription and translation are compartmentalized. I.e., transcription and translation are performed in separate reactors, which has the advantage that the reaction conditions for transcription and translation can be chosen and optimized separately. The separate reactors may be interconnected within a (semi)continuous process, enabling reaction control of individual reactions and overall yield optimization.

Another advantage of using separate but interconnected reconstituted systems for transcription and translation is that it opens up the possibility of introducing efficient recycling and regeneration streams, such that the concentration of necessary components for both reactions can better be controlled. This further ensures sustainability and reduces production costs, because enzymes and other components used in the reactions can be regenerated or recycled and reused. As such, the lifetime of the system reactions is no longer limited by the stability and amount of active and/or essential reaction components present in the system but can be extended by regenerating and/or recycling these components.

Furthermore, the platform allows for a generic set-up, requiring the replacement of DNA for production of peptides and proteins on demand. The newly developed methods and systems combine the use of a minimal enzymatic system, reaction compartmentalization and reuse of biomolecules within the synthesis. This enables from small to large scale peptide and protein synthesis with total reaction control, reduced timelines and production costs for therapeutic applications.

In a first aspect, the invention therefore provides a method for *in vitro* synthesis of a peptide or protein comprising:
a) transcribing a DNA into RNA in a first reaction container comprising the DNA and an enzyme capable of transcribing DNA into RNA,
b) introducing the RNA into a second reaction container, and
c) translating the RNA into a peptide or protein in the second reaction container,
wherein the first reaction container and the second reaction container are connected.

Also provided is a system for reconstituted cell-free protein synthesis, comprising:
- a first reaction container comprising a reaction solution comprising an enzyme capable of transcribing DNA into RNA and ribonucleotides, preferably nuceloside triphosphates (NTPs),
- a second reaction container comprising a reaction solution comprising ribosomes, tRNAs, amino acids, aminoacylated tRNAs, catalysts for tRNAs aminoacylation and/or translation factors for protein synthesis, and
- a conduit for conducting RNA from the first reaction container into the second reaction container.

Also provided is a method of the invention, which comprises the use of a system according to the invention. Also provided is a use of a system of the invention for the synthesis of a peptide or protein.

The terms "protein" and "peptide" refer to compounds comprising natural and/or non-natural amino acids linked together via peptide bonds. Typically, a peptide is defined as a compound that consists of 2 to about 50 amino acids, whereas a protein is made up of 50 or more amino acids. The peptide or protein that is synthesized is not limited with respect to the length thereof.

As used herein, "DNA" refers to any type of deoxyribonucleic acid. The term DNA includes recombinant and synthetic DNA as well as DNA, which is isolated from a natural source and cDNA. In some embodiments, the DNA is linear DNA. In some embodiments, the DNA is non-linear DNA, such as a DNA plasmid. The DNA is a DNA starting material for peptide or protein synthesis. The DNA encodes the peptide or protein that is or that is to be synthesized, but may include further nucleotides, and is herein also referred to as "DNA template". The DNA can be a double-stranded DNA or a single-stranded DNA, or a combination thereof. Most RNA polymerases transcribe double-stranded DNA into RNA, but some RNA polymerases transcribe single-stranded DNA, e.g., comprising a single-stranded promoter that is operably joined to a single-stranded DNA sequence, into RNA. Transcription of DNA gives rise to a single-stranded RNA molecule. The DNA is preferably, at least in part, double stranded.

The term "RNA" as used herein includes any type of ribonucleic acid. In preferred embodiments the RNA that is transcribed from DNA in the first reaction container(s) is mRNA.

The method of the invention is a cell-free peptide and protein synthesis method. Similarly, the system of the invention is a cell-free peptide and protein synthesis. Both the transcription reaction and the translation reaction are performed cell-free. This means that peptide or protein synthesis, i.e., transcription and translation, is not performed by living cells. Further, the method of the invention is preferably performed using a reconstituted cell-free protein synthesis system. Similarly, the system of the invention is preferably a reconstituted cell-free peptide and protein synthesis. As used herein "reconstituted" means that the system of the invention or used in accordance with the invention is reconstituted from individual or combined purified components that allow for the transcription of the DNA template and translation of the RNA into peptide / protein. In preferred embodiments, the reconstituted system is constituted by enzymes produced by recombinant expression. Hence, in preferred embodiments, the method is performed using a reconstituted cell-free protein synthesis system. In further preferred embodiments, the system of the invention is a reconstituted cell-free protein synthesis system. In particular, it means that no cell extracts are used in a method of the invention or present in a system of the invention. As used herein "cell extract" refers to a lysate or a cellular preparation from a cell without substantive isolation or purification step or steps, e.g., prepared by a physical or chemical disruption of the cell, and is also referred to as a "cell lysate". In particular, no cell extracts are used neither for transcription nor translation. I.e., a method of the invention is a cell extract-free method and the system of the invention or used in the invention is a cell extract-free system. However, the method and system of the invention may make use of components that are obtained from cell lysates, e.g., isolated or purified from cell lysates, e.g., ribosomes, tRNAs and/or enzymes produced in cells. A reconstituted cell-free protein synthesis system consists of several components from the cellular machinery, such as enzymes, ribosomes and tRNAs and additional components, such as NTPs and amino acids that are necessary for the protein synthesis. The system is supplemented with further reaction components such as salts and cofactors (e.g., magnesium ions) in an intent to mimic the cell environment for an efficient synthesis, and optionally additional components, such as NTPs and amino acids. In preferred embodiments, the components constituting the reconstituted cell-free protein synthesis system of the invention or used in accordance with the invention are purified components. Purified components can be individually purified components or purified combinations of individual components. As used herein "purified components" means that the individual components or combinations of components are specifically purified from a natural source, such as living cells (including cells used for recombinant expression of enzymes), or from a synthetic source. Combinations of individual components in particular consist of components of the same class, e.g., a mixture of different tRNAs and a mixture of different enzymes. The term "individually purified components" includes a mixture of individually purified components. I.e., the terms "purified components" and "individually purified components" includes a mixture of previously purified components. Thus, the purified components can be mixed before being added to a system or reaction container in accordance with the invention.

A method of the invention comprises the use of a first reaction container and a second reaction container. A system of the invention comprises a first reaction container and a second reaction container. In the first reaction container transcription of nucleic acid, in particular DNA, into RNA is performed. In the second reaction container, translation of RNA into a peptide or protein is performed.

Hence, in accordance with the present invention, the transcription and translation reactions, and therefore their corresponding reaction containers, are physically separated. However, the first reaction container wherein the nucleic acid is transcribed in RNA and the second reaction container wherein the RNA is translated into a peptide or protein are connected. Connection is preferably achieved by, at least, a conduit for conducting RNA from the first reaction container into the second reaction container, in particular from an outlet of the first reaction container into an inlet of the second reaction container.

The first reaction container, in particular when in use, comprises a DNA, and an enzyme capable of transcribing DNA into RNA. The DNA encodes the peptide or protein that is or that is to be synthesized. The DNA sequence of interest may be comprised in a larger DNA molecule. In some embodiments, the first reaction container does not comprise any DNA encoding a peptide or protein other than the DNA encoding the peptide or protein that is or that is to be synthesized. In preferred embodiments, the enzyme capable of transcribing DNA into RNA is an RNA polymerase. However, synthetic or engineered enzymes capable of transcribing DNA into RNA may also be used. RNA polymerases, which catalyze the synthesis of RNA from a DNA template, are well known in the art and any RNA polymerase, from a natural source or engineered, can be used in the present invention, in particular DNA dependent RNA polymerases. Examples of suitable RNA polymerases include, but are not limited to, T7 RNA polymerase, T3 RNA polymerase, K11 RNA polymerase and SP6 RNA polymerase. A suitable RNA polymerase can be selected by a person skilled in the art. In preferred embodiments, the first reaction container comprises the enzyme capable of transcribing DNA into RNA, preferably the RNA polymerase, as the only enzyme responsible of the transcription of DNA into RNA. In some embodiments, the first reaction container does not comprise any peptide or protein other than the enzyme capable of transcribing DNA into RNA, preferably the RNA polymerase.

The first reaction container further preferably comprises all components necessary for transcribing a DNA template into RNA. In particular, the first reaction container preferably comprises a reaction solution comprising ribonucleotides, preferably, nucleoside triphosphates (NTPs). The reaction solution is preferably a buffer solution that allows the synthesis of RNA from a DNA template, catalyzed by the enzyme capable of transcribing DNA into RNA. Suitable reaction solutions with additional reaction components required for a particular enzyme, e.g., RNA polymerase, to transcribe DNA into RNA are well known in the art and are commercially available or can be prepared from their commercially available constituting components. NTPs refer to a mixture of substrates required for the synthesis of RNA, in particular adenosine triphosphate (ATP), guanosine triphosphate (GTP), cytidine triphosphate (CTP) and uridine triphosphate (UTP).

In preferred embodiments, the first reaction container does not contain a cell extract. In further preferred embodiments, the first reaction container comprises purified components necessary for transcription of DNA into RNA, in particular the DNA template, enzyme capable of transcribing DNA into RNA and NTPs as defined herein. In further preferred embodiments, the first reaction container comprises a reaction solution comprising further reaction components such as buffer(s), salt(s) cofactor(s) (e.g., magnesium ions), reducing agents, crowding agents and/or other enzymes like RNAse inhibitor and pyrophosphatase. In some embodiments, the first reaction container comprises a reaction solution comprising buffer(s), salt(s) cofactor(s) (e.g., magnesium ions), and/or other enzymes like RNAse inhibitor and pyrophosphatase, as described herein.

In further preferred embodiments, the first reaction container essentially does not or does not contain components of the translation system, i.e., components that are necessary for translation of RNA into peptide or protein. In particular, the first reaction container preferably does not comprise ribosomes, tRNAs, amino acids, aminoacylated tRNAs, catalysts for tRNA aminoacylation or translation factors for protein synthesis.

In some embodiments, a method of the invention further comprises removing formed byproducts and/or refreshing reaction components such as small molecules, e.g., related to components of the reaction solution such as salt(s), NTPs or cofactor(s) that are consumed and/or transformed during transcription. This allows controlling the composition in the first reaction container. In further embodiments, the system of the invention or system used in a method of the invention additionally comprises a separation system integrated in or connected to the first reaction container, e.g., that allows filtration by size. Optionally such system may further comprise a conduit for conducting byproducts and/or waste products from the transcription reaction from the first reaction container, or an outlet thereof, optionally to a waste module, or an inlet thereof. Additionally or alternatively, such system may comprise a conduit for providing one or more reaction components and/or purified components necessary for transcription of DNA into RNA as defined herein above to the first reaction container or to an inlet thereof. In further embodiments, the liquid stream may be recycled without regeneration and/or adding new components.

The second reaction container, in particular when in use, preferably comprises components necessary for translating RNA into a peptide or protein. In preferred embodiments, the second reaction container comprises ribosomes, tRNAs, amino acids, aminoacylated tRNAs, catalysts for tRNAs aminoacylation, such as aminoacyl-tRNA synthetases or flexizymes, and/or translation factors for protein synthesis. In further preferred embodiments, the second reaction container comprises ribosomes, translation factors for protein synthesis, and further comprises tRNAs, or aminoacylated tRNAs. In further embodiments, the second reaction container additionally comprises catalysts for tRNAs aminoacylation, such as aminoacyl-tRNA synthetases, and amino acids. All these components and methods to obtain them are known in the art. Translation factors for protein synthesis include the translation initiation factors, elongation factors and release or termination factors. These factors and other components for the translation reaction can be purified from recombinant expression in cells or from lysates from prokaryotic cells, such as *E. coli,* but also from eukaryotic cells.

The second reaction container further preferably comprises a reaction solution comprising other reaction components, e.g., buffer(s), salt(s), cofactor(s), crowding agent(s), reducing agent(s) and/or other enzymes like RNAse inhibitor and pyrophosphatase. In some embodiments, the second reaction container comprises a reaction solution comprising buffer(s), salt(s) cofactor(s) (e.g., magnesium ions), and/or other enzymes like RNAse inhibitor and pyrophosphatase, as described herein. The reaction solution is preferably an aqueous buffer solution that allows for the synthesis of peptide(s) or protein(s) from RNA, catalyzed by the components of the translation system. Suitable compositions of the reaction solution are well known in the art and can be commercially available components.

In preferred embodiments, the second reaction container does not contain a cell extract. In further preferred embodiments, the second reaction container comprises the purified components necessary for translation of RNA, in particular ribosomes, tRNAs, amino acids, aminoacylated tRNAs, catalysts for tRNAs aminoacylation and/or translation factors for protein synthesis as defined herein. In preferred embodiments, the catalysts for tRNAs aminoacylation are aminoacyl-tRNA synthetases.

In preferred embodiments, the second reaction container essentially does not or does not contain components of the transcription system that are only necessary for transcription of DNA into RNA, in particular DNA template and/or enzyme capable of transcribing DNA into RNA. NTPs, or at least ATP and/or GTP are present in the second reaction container because they constitute the energy source that is consumed during the translation reaction. In addition, RNA degradation products, such as NDPs/NMPs, can be present. Hence, in particularly preferred embodiments, the second reaction container essentially does not or does not comprise DNA template and enzyme capable of transcribing DNA into RNA.

The first and/or second reaction container may further comprise components other than the components necessary for transcription (for the first reaction container) and the components necessary for translation (for the second reaction container). Examples of such other components include enzymes for regenerating energy in the reaction system, such as creatine kinase, myokinase, nucleoside diphosphate kinase and/or polyphosphate kinase (PPK) enzymes, e.g., PPK2. The first and/or second reaction container may further comprise an enzyme or enzymes for hydrolysing pyrophosphate formed during the transcription or translation, in particular during the aminoacylation of tRNAs, reaction, into inorganic phosphate.

In preferred embodiments, the DNA template is immobilized on a solid support. Hence, in preferred embodiments, the first reaction container comprises DNA or part thereof immobilized on a solid support. As used herein "immobilized on a solid support" indicates that the DNA is attached to a solid support.

Immobilization of DNA can be achieved in multiple ways, which are commonly known in the art. Similarly, suitable solid supports for immobilization of DNA are commonly known in the art. For example, streptavidin-biotin immobilization, whereby biotinylated DNA is immobilized on a streptavidin-coated support. However, a skilled person is well capable of identifying and selecting other methods for DNA immobilization. Suitable solid supports are for instance columns or beads, such as (nano- or micro-)particles, (nano- or micro-)fibres, (nano- or micro-)plates and (nano- or micro-)rods, e.g., inorganic (e.g., magnetic or silica) or polymer (e.g., Sepharose) columns or beads such as (nano- or micro-)particles, (nano- or micro-)fibres and (nano- or micro-)rods incorporating accessible functional groups. In the Examples herein, streptavidin surface-coated beads are used.

The use of DNA immobilized on a solid support enables separation of the DNA and the transcribed RNA, which is not immobilized. This allows for a continuous or semi-continuous process. In particular, the transcribed RNA and enzyme capable of transcribing DNA into RNA, preferably RNA polymerase, are comprised within the liquid, aqueous phase formed by the reaction solution in the first reaction container, in such a way that the liquid stream with the transcribed RNA and enzyme capable of transcribing DNA into RNA can be separated from the immobilized DNA. This allows introducing the isolated RNA in the second reaction container, wherein its translation into peptide(s) or protein(s) takes place. Moreover, it may allow recycling or reuse of the DNA template in the transcription reaction container while also avoiding additional need of DNAse treatments.

In preferred embodiments, a method of the invention therefore comprises separating the DNA and the RNA, prior to introducing the RNA into the second reaction container. In further preferred embodiments, a method of the invention therefore comprises separating the DNA and the RNA prior to introducing the RNA into the second reaction container. In further preferred embodiments, a method of the invention therefore comprises separating the DNA and the RNA and enzyme capable of transcribing DNA into RNA prior to introducing the RNA into the second reaction container. This can for instance be achieved by removing a liquid stream comprising the RNA from the first reaction container comprising the immobilized DNA. The liquid stream comprises the reaction solution or part thereof from the first reaction container, including the RNA. The liquid stream comprising the RNA can be directly introduced into the second reaction container or, alternatively, the liquid stream can be processed before introduction into the second reaction container. As used herein "processed" means that one or more treatments are performed on the liquid stream comprising the RNA, such as separation of RNA and the enzyme capable of transcribing DNA into RNA, preferably RNA polymerase, purification of the RNA, elution of the enzyme, preferably RNA polymerase, and/or reaction solution exchange.

In preferred embodiments, the RNA and enzyme capable of transcribing DNA into RNA are additionally separated prior to introducing the RNA into the second reaction container. This has the advantage that the enzyme capable of transcribing DNA into RNA is not present in the second reaction container where the translation reaction takes place and where enzyme capable of transcribing DNA into RNA is not being used. In addition, separation of the enzyme capable of transcribing DNA into RNA from the liquid stream that is introduced into the second reaction container may allow recycling thereof. I.e., after separation, the RNA polymerase can be reintroduced into the first reaction container. NTPs may still be present in the liquid stream comprising the RNA but not the enzyme capable of transcribing DNA into RNA, without having a detrimental effect on the translation reaction. Hence, in preferred embodiments, a method of the invention further comprises separating the enzyme capable of transcribing DNA into RNA, preferably RNA polymerase, and the RNA prior to introducing the RNA into the second reaction container. In further preferred embodiments, the enzyme capable of transcribing DNA into RNA, preferably RNA polymerase, that is separated from the RNA is reintroduced into the first reaction container. If desired, NTPs can be separated from the RNA, e.g., based on size or affinity chromatography.

In preferred embodiments, separating the enzyme capable of transcribing DNA into RNA and the RNA is performed in a separation module. Such separation module may be located between the first and second reaction containers. Separation of RNA and the enzyme can be achieved by any method known in the art. As an example, separation of RNA and enzyme for instance may be done based on size, e.g., using size exclusion chromatography, or using affinity chromatography. Alternatively, a labelled enzyme, e.g., RNA polymerase, can be used, also referred to as a tagged enzyme, e.g., tagged RNA polymerase, and separation can be based on capturing the labelled enzyme by affinity chromatography. Hence, in some embodiments, the enzyme is labelled, preferably with a label, also referred to as a tag, that allows capturing of the enzyme.

In preferred embodiments, a method or system of the invention therefore further comprises a separation module for separating the enzyme capable of transcribing DNA into RNA, preferably RNA polymerase, and RNA. The separation module is preferably located between the first and second reaction containers. I.e., the separation module preferably interrupts the conduit for conducting RNA from the first reaction container to the second reaction container. I.e., in a system of the invention the conduit for conducting RNA from the first reaction container to the second reaction container preferably comprises a separation module for separating RNA and the enzyme capable of transcribing DNA into RNA. The conduit preferably comprises a first conduit part for conducting RNA and enzyme capable of transcribing DNA into RNA from the first reaction container, or an outlet thereof, to the separation module for separating RNA and the enzyme capable of transcribing DNA into RNA, or an inlet thereof, and a second conduit part for conducting RNA from the separation module for separating RNA and the enzyme capable of transcribing DNA into RNA, or an outlet thereof, to the second reaction container, or an inlet thereof.

In further preferred embodiments, the RNA is separated from both the DNA and the enzyme capable of transcribing DNA into RNA before the RNA is introduced into the second reaction container, e.g., by first separating a liquid stream comprising RNA and the enzyme from immobilized DNA and subsequently separating RNA from the enzyme.

Following separation of RNA and enzyme capable of transcribing DNA into RNA, the method of the invention may comprise a step of elution of the RNA or enzyme and adjusting the reaction solution containing the RNA or enzyme e.g., with a suitable buffer solution. The final solution containing RNA is suitable for the translation of the RNA in the second reaction container. The final solution containing the enzyme capable of transcribing DNA into RNA is suitable for reuse of the enzyme in the first reaction container.

In some embodiments, the system of the invention or used in the invention further comprises a compartment for further purification, of RNA and/or enzyme capable of transcribing DNA into RNA, and/or reaction solution exchange, in particular of the buffer that comprises the RNA transcribed in the first reaction container and/or the buffer that comprises the enzyme capable of transcribing DNA into RNA. This would guarantee the suitability of the RNA and the enzyme, for instance after elution, before being transferred into the second and first container, respectively. Hence, the compartment for further purification and/or reaction solution exchange is preferably encompassed in the separation module in which RNA and enzyme capable of transcribing DNA into RNA are separated, or is located downstream of the separation module.

The separation of DNA template, RNA and enzyme capable of transcribing DNA into RNA, preferably RNA polymerase, also allows performing the method of the invention in a continuous or semi-continuous manner whereby the RNA is continuously or semi-continuously introduced into the second reaction container and the enzyme that is separated from the RNA may be continuously or semi-continuously reintroduced into the first reaction container. As used herein, a continuous manner refers to a process wherein the components, i.e., enzyme capable of transcribing DNA into RNA and recycled enzyme, NTPs, and/or reaction solution comprising other reaction components, e.g., cofactors, are continuously added while products such as RNA are continuously removed. A semi-continuous process refers to a process wherein components, i.e., enzyme capable of transcribing DNA into RNA and recycled enzyme, NTPs and/or reaction solution comprising other reaction components, e.g., cofactors, can be periodically added and/or products such as RNA can be periodically removed. In preferred embodiments, a method of the invention is performed in a continuous or semi-continuous manner, more preferably a semi-continuous manner.

In some preferred embodiments, the method is performed in a continuous or semi-continuous manner, whereby the RNA is continuously or semi-continuously introduced into the second reaction container and/or the enzyme capable of transcribing DNA into RNA that is separated from the RNA is continuously or semi-continuously reintroduced into the first reaction container.

Separation of the DNA template, RNA and enzyme capable of transcribing DNA into RNA, preferably RNA polymerase, and/or reusing the enzyme capable of transcribing DNA into RNA also allows for a simultaneous or in part simultaneous (i.e., overlapping) performance of the transcription reaction in the first reaction container and the translation reaction in the second reaction container. Hence, in preferred embodiments, the transcription reaction in the first reaction container and the translation reaction in the second reaction container are performed such that they at least overlap in time. In further preferred embodiments, the method is performed in a continuous or semi-continuous manner, whereby the RNA is continuously or semi-continuously introduced into the second reaction container and enzyme that is separated from the RNA is continuously or semi-continuously reintroduced into the first reaction container. More preferably, the method is performed in a semi-continuous manner, whereby the RNA is semi-continuously introduced into the second reaction container and enzyme that is separated from the RNA may be semi-continuously reintroduced into the first reaction container.

In some embodiments, more than one second reaction container is used. Hence provided is a method of the invention, wherein the RNA is introduced in a plurality of second reaction containers, and wherein the first reaction container and the plurality of second reaction containers are connected. Also provided is therefore a system for reconstituted cell-free protein synthesis according to the invention comprising a plurality of second reaction containers and a plurality of conduits for conducting RNA from the first reaction container into the second reaction containers. The plurality of second reaction containers are independently a second reaction container as defined and described herein. Hence, the different second reaction containers of the plurality of second reaction containers may be the same or different, e.g. in terms of components comprised therein. As used herein the term "plurality" refers to more than one. In some embodiments the plurality of second reaction containers may range from 2 to 100 or more second reaction containers.

In some embodiments, more than one first reaction container is used. Hence, provided is a method of the invention, wherein the DNA is transcribed into RNA in a plurality of first reaction containers comprising the DNA and an enzyme capable of transcribing DNA into RNA and wherein the plurality of first reaction containers and the second reaction container are connected. Also provided is therefore a system for reconstituted cell-free protein synthesis according to the invention comprising a plurality of first reaction containers and a plurality of conduits for conducting RNA from the first reaction containers into the second reaction container. The plurality of first reaction containers are independently a first reaction container as defined and described herein. Hence, the different first reaction containers of the plurality of first reaction containers may be the same or different, e.g. in terms of components comprised therein. As used herein the term "plurality" refers to more than one. In preferred embodiments the plurality of first reaction containers may range from 2 to 100 or more first reaction containers.

In some embodiments, more than one first reaction container and more than one second reaction container are used. Hence provided is a method of the invention, wherein the DNA is transcribed into RNA in a plurality of first reaction containers comprising the DNA and an enzyme capable of transcribing DNA into RNA, wherein the RNA is introduced in a plurality of second reaction containers and wherein the plurality of first reaction containers and the plurality of second reaction containers are connected. Also provided is therefore a system for reconstituted cell-free protein synthesis according to the invention comprising a plurality of first reaction containers and a plurality of second reaction containers and a plurality of conduits for conducting RNA from the first reaction containers into the second reaction containers.

In some embodiments, a method of the invention further comprises aminoacylation of tRNAs in an aminoacylation module, and preferably further comprises introducing the aminoacylated-tRNAs (aa-tRNAs) into the second reaction container.

In some embodiments, a method of the invention comprises regeneration of energy sources in an energy regeneration module, and introducing the regenerated energy sources into the second reaction container. In preferred embodiments, the energy source or sources are phosphate containing energy sources and regeneration comprises phosphorylation of the energy source. I.e., phosphate groups are significantly consumed during translation and the energy source can be refreshed or regenerated, e.g., by phosphorylation. In preferred embodiments, the energy source is one or more nucleotide triphosphates, preferably ATP and/or GTP. In the transcription reaction, ATP and GTP are mostly consumed as constituting substrates during RNA synthesis; in translation ATP is mainly required for the aminoacylation of tRNAs, while major GTP consumption is related to the peptide bond formation, and may be used for folding purposes in the presence of several chaperones. ATP can be used to regenerate NTPs like GTP. Moreover, both energy substrates can be regenerated by different phosphorylation alternatives, e.g., using a second phosphate donor, like creatine phosphate and further enzyme or enzymes, and/or a bifunctional kinase like PPK2 and polyphosphate as energy source. Hence, in further preferred embodiments, the energy regeneration module comprises a further enzyme or enzymes for phosphorylation of dephosphorylated NTPs.

The peptide or protein synthesized with a method of the invention can be isolated from the reaction mixture in the second reaction container. In preferred embodiments, a method of the invention therefore comprises isolating the peptide or protein, in particular isolating the peptide or protein from the second reaction container. Isolation of the peptide and protein can be performed with methods known in the art for isolating or purifying proteins from cell-free, cell or cell extract based *in vitro* protein synthesis systems. A person skilled in the art is well capable of selecting a suitable purification method.

As an example, isolation of peptides or proteins can for instance be done based on size, e.g., using size exclusion chromatography, or using an affinity chromatography. If desired, the protein components constituting the translation system can be labeled to include a tag in order to separate them from the unlabeled peptide or protein that is synthesized in the second reaction container, e.g., as described in WO02053582, which is incorporated herein by reference.

As another example, peptides or proteins that are synthesized in the second reaction container can be immobilized on a matrix during the translation process, e.g., by using a matrix that specifically binds peptides or proteins by hydrophobic, hydrophilic, antigen/antibody, or ionic interactions, e.g., the matrix contains anion or cation exchange material, e.g., as described in US2005227312A1, which is incorporated herein by reference. Alternatively, the peptide or protein can be synthesized as part of a fusion peptide or protein, wherein the fusion partner of the peptide or protein specifically binds to the matrix.

In some embodiments, a method or system of the invention further comprises an isolation container for isolation of the peptide or protein of interest. This container is preferably located downstream of the second reaction container. It preferably comprises means for isolation and/or purification of the peptide and/or protein. In further embodiments, the system comprises a conduit for conducting the peptide or protein, and optionally other reaction components, from the second reaction container or an outlet thereof to the isolation container. Furthermore, the system may comprise a conduit for conducting the isolated and/or purified peptide or protein from the isolation container or an outlet thereof. Optionally, the system comprises a further conduit for conducting reaction components from the isolation container or an outlet thereof to the second reaction container or an inlet thereof. In this way, recirculation of reaction components of the translation reaction is possible. That would allow for a continuous or semi-continuous process for production of peptides or proteins with further reuse of the translation machinery.

In some embodiments, the DNA is immobilized on a support in the form of a cartridge. As used herein a "cartridge" refers to a support that is arranged to be inserted to and removed from the first reaction container. This allows for an easy replacement of DNA from and into the first reaction container. Non-limiting examples of cartridges are a packed column, a porous structure or a solid structure with the shape of a cylinder, a plate or a column containing stacked plates or beads with immobilized DNA on their surface. In further preferred embodiments, the first reaction container is arranged such that a cartridge comprising the immobilized DNA can be inserted to and removed from the container. This allows reusing the first reaction container and optionally the components of the transcription reaction and/or the entire system for the synthesis of different peptides and/or proteins. In order to synthesize different peptides and/or proteins, the cartridge with immobilized DNA that encodes a first peptide or protein of interest can be replaced by a different cartridge with a different immobilized DNA that encodes a second peptide or protein of interest, e.g., following synthesis of the first peptide or protein of interest.

In a system or method of the invention, the first reaction container therefore preferably comprises an access for insertion of a solid support comprising DNA immobilized thereto into the first reaction container such that the immobilized DNA is in contact with the reaction solution and further reaction components, comprising the enzyme capable of transcribing DNA into RNA, preferably RNA polymerase, and NTPs. The first reaction container wherein such cartridge is inserted is preferably arranged such that a cartridge is inserted in a leak-proof manner. I.e., once the cartridge is inserted and the first reaction container comprises the reaction solution and reaction components of the transcription system, no leakage of the reaction mixture occurs.

Optionally other reaction components for both the transcription and the translation reaction, such as enzymes, can be immobilized on a support, e.g., a support in the form of a cartridge as defined herein above. This allows for an easy replacement of such components from and into the first and/or second reaction container.

A system of the invention comprises a conduit for conducting RNA from the first reaction container or an outlet thereof to the second reaction container or an inlet thereof. In some embodiments, the conduit comprises a part for transferring a liquid, aqueous stream comprising all components present in the first reaction container, with the exception of immobilized DNA, and introducing this liquid, aqueous stream into a separation module or an inlet thereof for separating the RNA and enzyme capable of transcribing DNA into RNA.

Hence, in preferred embodiments, part of the conduit for conducting RNA from the first reaction container into the second reaction container is for conducting RNA from the first reaction container, or an outlet thereof, to a separation module, or an inlet thereof, for separating the RNA and enzyme capable of transcribing DNA into RNA. In further preferred embodiments, part of the conduit for conducting RNA from the first reaction container into the second reaction container is for conducting RNA from the separation module for separating the RNA and enzyme capable of transcribing DNA into RNA, or an outlet thereof, to the second reaction container, or an inlet thereof.

In some embodiments, a system of the invention or used in the invention further comprises a compartment for further purification of RNA and/or enzyme capable of transcribing DNA into RNA, preferably RNA polymerase, and/or for exchange of the solution, in particular of the solution that comprises the RNA transcribed in the first reaction container and/or the solution that comprises the enzyme capable of transcribing DNA into RNA. Exchange into a suitable solution would guarantee the suitability of the RNA and enzyme, for instance after elution from a chromatography column, before being transferred into the second and first container, respectively. Hence, the compartment for further purification and/or solution exchange is preferably encompassed in the separation module in which the RNA and enzyme are separated, or is located downstream of the separation module and upstream of the second and/or first reaction containers, respectively.

In preferred embodiments, the first reaction container comprises an inlet for the enzyme capable of transcribing DNA into RNA, preferably RNA polymerase, NTPs and/or reaction solution comprising other reaction components, e.g., cofactors (magnesium ions), and/or an inlet for the enzyme capable of transcribing DNA into RNA, an inlet for NTPs and an inlet for reaction solution containing additional reaction components.

In some embodiments, the first reaction container comprises an inlet for refreshing the reaction solution, such as the buffer and other reaction components such as NTPs and cofactors, and an outlet for reaction by-products, e.g., pyrophosphate or precipitates. The latter inlet and/or outlets may additionally be connected to a control module to control the reaction composition. The temporary confinement of some of the remaining reaction components in the first reaction container, with respect to the refreshed ones, may be achieved by strategies of immobilization and/or (ultra)filtration, e.g., using a membrane.

In some preferred embodiments, the second reaction container comprises an inlet for aa-tRNAs.

In some preferred embodiments, the second reaction container comprises an outlet for tRNAs.

In some preferred embodiments, the system comprises a conduit for conducting tRNAs from the second reaction container or an outlet thereof to an aminoacylation module for aminoacylation of tRNAs or an inlet thereof. In further preferred embodiments, the system comprises a conduit for conducting aa-tRNAs from the aminoacylation module or an outlet thereof to the second reaction container or an inlet thereof.

In some embodiments, the second reaction container comprises an outlet for reaction byproducts like dephosphorylated NTPs, in particular dephosphorylated ATP and/or GTP, pyruvate phosphate, inorganic phosphate and/or precipitates. Optionally such system may further comprise a conduit for conducting byproducts and/or waste products from the second reaction container, or an outlet thereof, optionally to a waste module, or an inlet thereof.

In some embodiments, the second reaction container comprises an inlet for refreshment of reaction components such as amino acids, cofactors sources, like magnesium, and/or energy sources like NTPs, in particular ATP and/or GTP, or precursors for the regeneration thereof.

In some embodiments, the system comprises a conduit for conducting reaction components and reaction solution from the second reaction container or an outlet thereof to a refreshment module for refreshment of reaction components. In further embodiments, the system comprises a conduit for conducting refreshed reaction components, e.g., cofactors, energy sources, amino acids and/or reaction solution comprising additional reaction components from the refreshment container or an outlet thereof to the second reaction container or an inlet thereof. Temporary confinement of the remaining reaction components in the second reaction container, with respect to the refreshed ones, may be achieved by strategies of immobilization and/or (ultra)filtration, e.g., using a membrane.

In some embodiments, the second reaction container and/or the aminoacylation module comprises an outlet for dephosphorylated energy sources, in particular dephosphorylated ATP and/or GTP, e.g., ADP, AMP, GDP and/or GMP.

In some embodiments, the second reaction container and/or the aminoacylation module comprises an inlet for amino acids and/or energy sources, in particular NTPs, in particular ATP and/or GTP.

In preferred embodiments, the system comprises a conduit for conducting dephosphorylated energy sources, in particular dephosphorylated ATP and/or GTP, e.g., ADP, AMP, GDP and/or GMP, from the second reaction container and/or the aminoacylation module or an outlet thereof to a separate reaction container for energy regeneration, in particular phosphorylation of dephosphorylated energy sources, in particular dephosphorylated ATP and/or GTP, e.g., ADP, AMP, GDP and/or GMP, or an inlet thereof. In further preferred embodiments, the system comprises a conduit for conducting NTPs as energy sources, in particular ATP and/or GTP from the energy regeneration module, in particular phosphorylation of dephosphorylated energy sources, in particular dephosphorylated ATP and/or GTP, e.g. ADP, AMP, GDP and/or GMP, or an outlet thereof to the second reaction container and/or the aminoacylation module or an inlet thereof.

Also provided is a use of a system of the invention for *in vitro* synthesis of a peptide or protein, preferably with a method according to the invention. Also provided is a method of the invention, which comprises the use of a system according to the invention, in particular, wherein transcribing a DNA into RNA and translating the RNA into a peptide or protein are performed with a system of the invention.

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.

The invention will be explained in more detail in the following, non-limiting examples.

### Brief description of the drawings

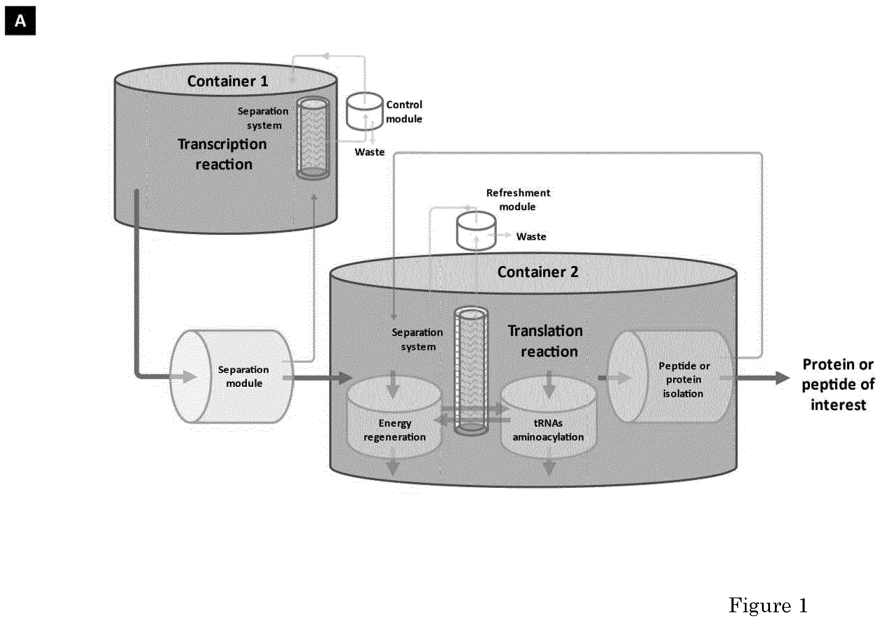

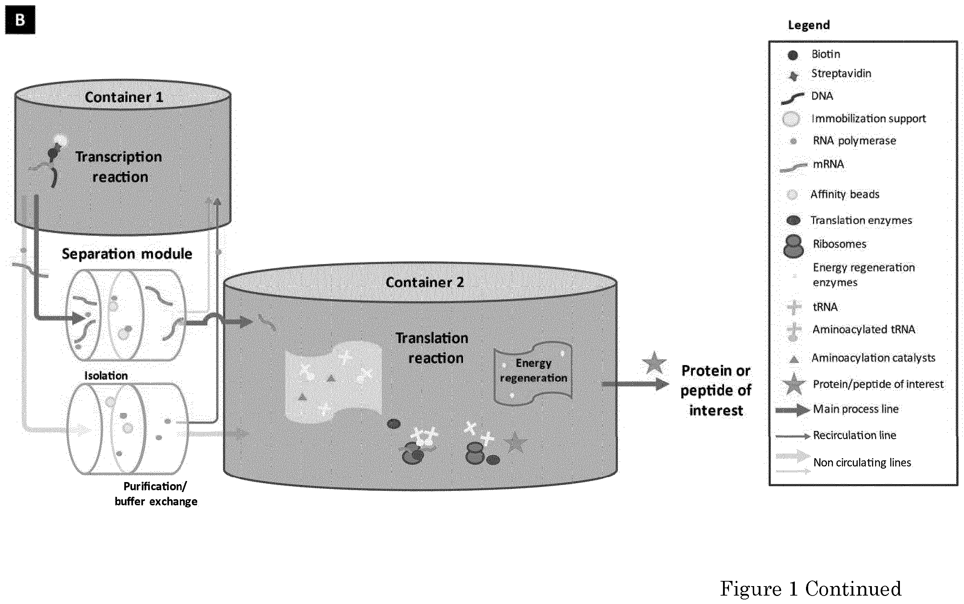

Figure 1: A,B. Schematic overview of exemplary methods and systems of the invention.

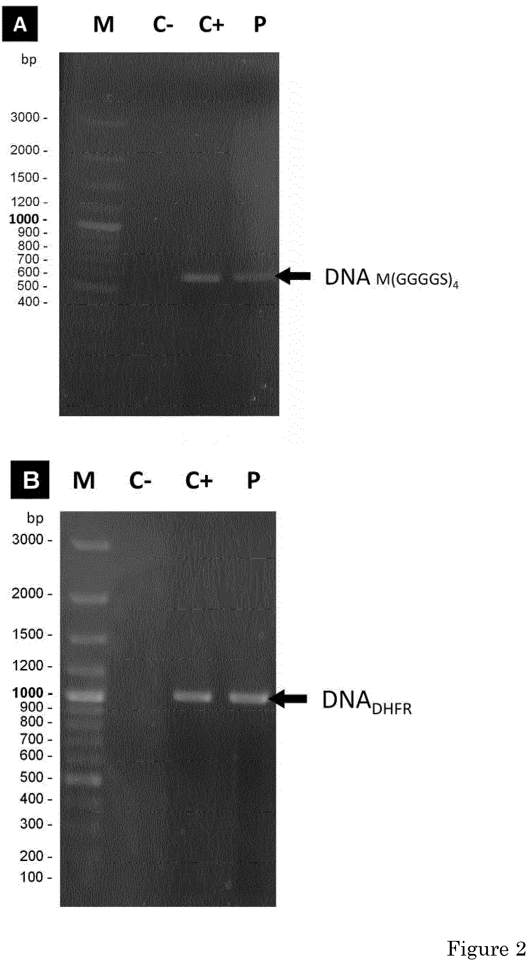

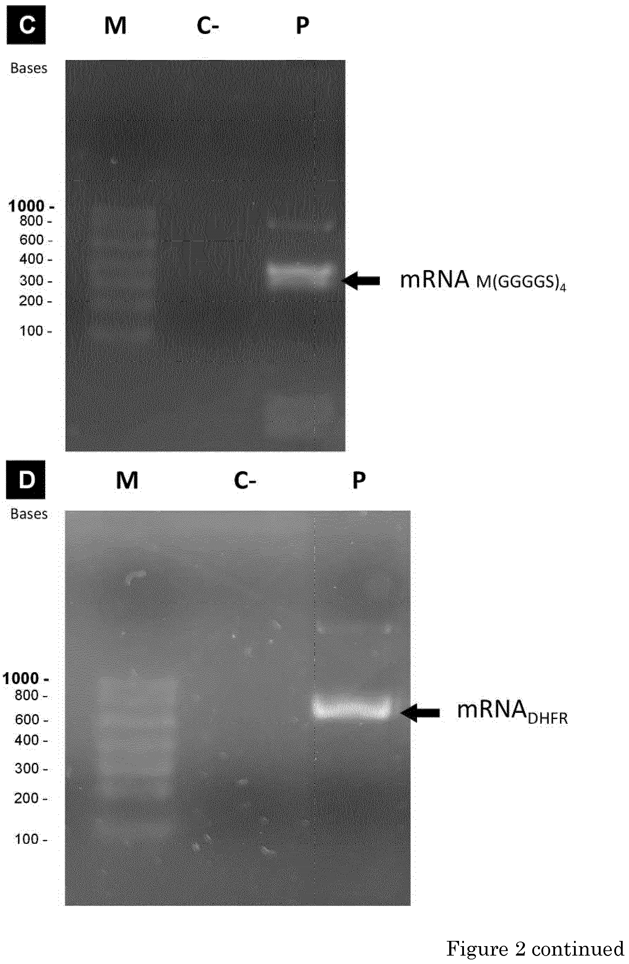

Figure 2: Agarose gel depicting 5'-biotinylated DNA (2A, 2B) and RNA (2C, 2D) products prepared by polymerase chain reaction (PCR) and *in vitro* transcription using immobilized DNA, respectively. The products of interest are encoding for a peptide (M(GGGGS)₄) (2A, 2C) and a protein, dihydrofolate reductase (DHFR) (2B, 2D), and are indicated with arrows. M = Marker; C- = Negative control; C+ = Positive control, being the linear DNA sequence of reference; P = Study sample containing the aimed products.

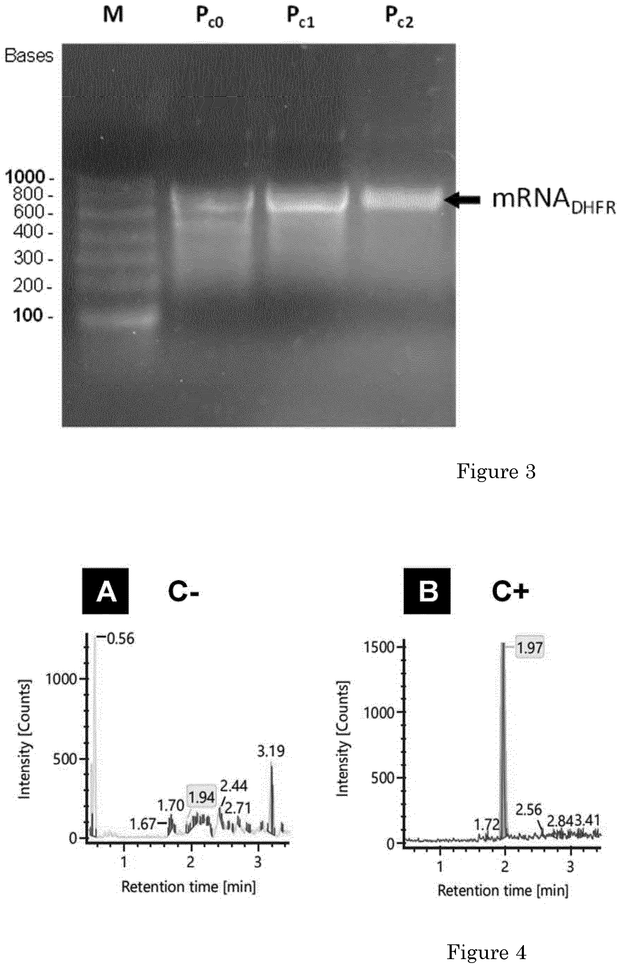

Figure 3: Agarose gel depicting the RNA encoding for DHFR, produced after 1 h of *in vitro* transcription reaction using 5'-biotinylated DNA immobilized on magnetic beads and by recycling a His-tagged T7 RNA polymerase. M = Marker; P_{c0}= RNA product from transcription reaction prior to enzymatic recycling; P_{c1} and P_{c2} = RNA products from transcription reactions after one and two cycles of enzymatic recycling, respectively.

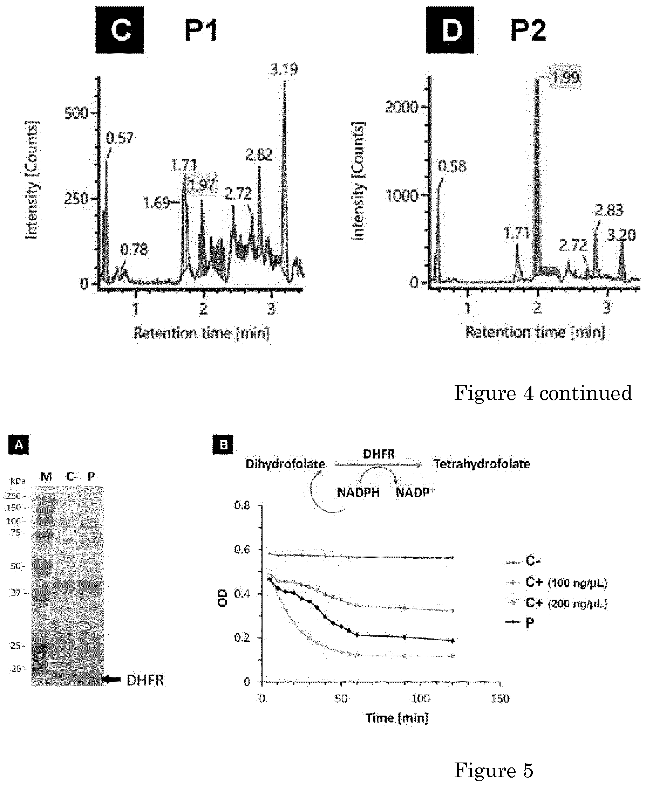

Figure 4: High Resolution Mass Spectroscopy (HRMS) chromatograms revealing the production of M(GGGGS)₄ as a test peptide: 4A (C-) = Negative control consisting of the PURE system depleted of T7 RNA polymerase, the ΔT7 PURE system, incubated without DNA nor mRNA template; 4B (C+) = Positive control, consisting of a standard reference solution containing 10 ng/mL of the synthetic peptide in MilliQ water; 4C (P1) and 4D (P2) = Products from the uncoupled in *vitro* transcription (3 h) and translation (3 h) reactions, with transfer to the ΔT7 PURE system of 1 µg (4C) and 6 µg (4D) of RNA produced by transcription of 5'-biotinylated DNA templates immobilized on magnetic beads.
Figure 5: Gel prepared by sodium dodecyl-sulfate polyacrylamide gel electrophoresis (SDS-PAGE) (5A) revealing the *in vitro* production of DHFR by an uncoupled transcription (3 h) reaction using 5'-biotinylated DNA immobilized on magnetic beads followed by RNA purification and translation (3 h) using the PURE system. The products were characterized upon completion of the translation reaction without further purification. M = Marker, C- = Negative control, consisting of the product from the PURE system incubated without DNA nor mRNA template; P = ΔT7 PURE product, containing the produced DHFR as the protein of interest. Evolution of absorbance versus time observed in the DHFR activity assay test (5B): C- = Negative control, consisting of the product from the PURE system without DNA nor mRNA template; C₊ = Standard sample, containing commercial DHFR as a reference at known concentrations of 100 or 200 ng/µL; P = Product from the ΔT7 PURE system fed with 25 µg RNA produced by *in vitro* transcription with 5'-biotinylated DNA immobilized on magnetic beads, containing the produced DHFR as the protein of interest.

### Examples

### Example 1: In vitro transcription with immobilized DNA

### 1. 1. DNA biotinylation

Biotinylated linear DNA templates were prepared by polymerase chain reaction (PCR) using 0.5 µM of biotinylated forward or reverse primers containing a triethylene glycol (TEG) spacer, and corresponding non-biotinylated reverse or forward primers from Eurofins Genomics. The DNA control plasmid from the PURE kit (New England Biolabs (NEB)), encoding for dihydrofolate reductase (DHFR) protein, and a linear template from Eurofin Genomics, encoding for a M(GGGGS)₄ peptide, were used as templates (2-250 pg/µL). Primers and DNA templates were combined in 5xPhusion High Fidelity buffer (21.5% v/v) aqueous solutions containing DMSO (3.2% v/v), 1 U of Phusion Hot Start II DNA polymerase and deoxynucleotide triphosphates (0.2 mM) from Thermo Fisher Scientific. Thermal cycling reactions were initiated by 30 s at 98 °C, followed by 30 cycles of 10 s at 98 °C, 30 s at 55 °C and 45 s at 72 °C, and completed by a final elongation phase of 5 min at 72 °C, before cooling down at 4 °C. Post-amplification, the PCR products were purified using PureLink PCR columns according to the manufacturer's procedures, as provided by Invitrogen. The resulting biotinylated double-stranded DNA templates were characterized by 1% agarose gel electrophoresis run at 100 V for 1 h, using a Topvision Agarose and TAE buffer from Thermo Fisher Scientific, and a DNA stain G from Serva Electrophoresis GmbH.

### 1.2. DNA immobilization

Biotinylated DNA (5'- or 3'-) templates were immobilized on the surface of streptavidin-coated magnetic beads, Dynabeads^{™}M-280 Streptavidin beads from Invitrogen, according to the manufacturer's procedures. The beads are provided as a 10 mg/mL suspension with an average binding capacity of 10 µg dsDNA/mg of beads. Thus, ratios of 1-3 µg of DNA template per 10 µL of suspension were used for immobilization purposes. Prior to immobilization, the beads were washed twice with 100 mM NaOH and 50 mM NaCl, once with 100 mM NaCl, and twice with a 5 mM Tris-HCl (pH 7.5), 0.5 mM ethylenediaminetetraacetic acid (EDTA) and 1 M NaCl buffer solution. After each washing step, the beads were isolated with the aid of a magnet, while the supernatant was discarded. Further, the beads were redispersed in 5 mM Tris-HCl (pH 7.5), 0.5 mM EDTA and 1 M NaCl buffer solution and incubated at room temperature with biotinylated PCR products for 15-30 min and under orbital shaking at 200 rpm. The samples were washed twice with a 5 mM Tris-HCl (pH 7.5), 0.5 mM EDTA and 1 M NaCl buffer solution, and once with 40 mM HEPES. The resulting immobilized DNA templates were resuspended either in 40 mM HEPES solution or MilliQ water and stored at 4 °C before use.

### 1.3. In vitro transcription

*In vitro* transcription was performed, as adapted from M. M. K. Hansen et al., ACS Synth. Biol. 2016, 5, 1433-1440, in aqueous solutions consisting of a 40 mM 4-(2-hydroxyethyl)-1-piperazineethanesulfonic acid (HEPES; pH 8.0) buffer, 10-40 mM magnesium chloride or magnesium acetate, 3.7 mM 1,4-dithiothreitol (DTT), 1 mM spermidine, 5 mM guanosine monophosphate (GMP) and 2-10 mM of individual nucleotide triphosphates (ATP, GTP, CTP and UTP) from Merck. Moreover, 1 U/µL Ribolock RNase inhibitor and 0.63-8.82 U/µL T7 RNA polymerase from Thermo Fisher Scientific were used as transcription enzymes, incubated with immobilized DNA templates (1.5-75 nM). For that aim, 50-500 µL reactions were carried out at 37 °C for 0.5-24 h, in low-binding or standard Eppendorf tubes, with or without orbital shaking at 200 rpm. Upon completion of the reaction, the samples were cooled down on ice and the DNA-coated magnetic beads were isolated by applying an external magnetic field for 2 min. Aliquots of the recovered supernatant, containing the synthesized mRNA, were purified according to the manufacturer's protocol using QIAGEN RNeasy kit or Monarch^{®} RNA Cleanup Columns (NEB). DNase I (Invitrogen/Ambion) treatment was performed, either before or during RNA purification, for 15 min at 37 °C. The isolated RNA was eluted in 20-50 µL of RNase-free water. Purification of RNA produced in larger transcription volumes (e.g., 500 µL) was accomplished by dividing the reaction solution in two aliquots that were subsequently loaded into two purification columns in multiple steps. In brief, the 50 µL eluate from the first column was further used for elution of the second column. This strategy enabled the adequate concentration of RNA samples for subsequent translation. The resulting RNA samples were run on 2% agarose gels for product characterization. Moreover, the DNA and RNA yields respectively achieved by PCR and *in vitro* transcription were determined by absorbance measurements with a NanoDrop One/OneC spectrophotometer (Thermo Fischer Scientific) or a Synergy HT Microplate reader (BioTek).

### 1.4 Results

Double-stranded biotinylated DNA linear templates encoding for M(GGGGS)₄, as a test peptide, and dihydrofolate reductase (DHFR), as a model protein, were prepared by PCR using biotin-TEG-primers. The theoretical length of 610 bp and 1024 bp of the corresponding PCR products is confirmed in the agarose gels depicted in Figure 2A (M(GGGGS)₄) and Figure 2B (DHFR). The biotinylated DNA was immobilized on streptavidin-coated magnetic beads and further used for *in vitro* transcription reactions. The analysis of the RNA products achieved after 3 h of incubation, as shown in the agarose gels from Figure 2C (M(GGGGS)₄) and Figure 2D (DHFR), reveal the successful formation of the product of interest, as the lower band observed in each gel. Furthermore, it is noticeable the presence of larger, run-off, translatable mRNA constructs, possibly resulting from a low recognition efficiency of the termination sequence by the T7 RNA polymerase. The upper RNA band corresponds to non-translatable constructs, possibly transcription errors from the T7 RNA polymerase, as previously reported (F.J. Triana-Alonso et al., J. Biol. Chem. 2008, 270, 6298-6307; X. Mu et al., Nucleic Acids Res. 2018, 46, s 5239-5249). Determination of the DNA immobilization performance by quantifying the free DNA remaining in the supernatant further enables controlling the input of DNA in the transcription reaction and overall, increasing the process robustness.

### Example 2: Recycling and reuse of enzymes

### 2.1. Recycling of T7 RNA polymerase

A His-tagged T7 RNA polymerase, supplied by NEB as part of a customized PURExpress kit depleted of this enzyme, was reused in successive *in vitro* transcription cycles of 1 or 3 h. Transcription reactions were performed according to Example 1, using reaction volumes of 50-200 µL. After each cycle, the immobilized DNA was isolated from solution with the aid of a magnet. The recovered solution was incubated in the presence of Dynabeads^{™} His-Tag Isolation and Pulldown beads (Invitrogen) in an orbital shaker for 10-20 min at room temperature. Before being added to a freshly prepared transcription reaction solution, the beads were washed according to the manufacturer's protocol, and dissolved in a 50-100 mM sodium-phosphate (pH 8.0) buffer solution containing 300-600 mM NaCl and 0.01-0.02% Tween-20. Alternatively, 50 mM Tris-HCl (pH 8.0), 100 mM NaCl and 1 mM DTT or 40 mM HEPES (pH 8.0), 40 mM MgCl₂, 3.7 mM DTT and 1 mM spermidine were used as immobilization solutions. Equivalent volumes of washed beads and reaction solutions were used for immobilization purposes, accounting for 1-20 µL of the commercial suspension of beads per sample. After incubation, the beads were isolated with a magnet and the supernatant was transferred to RNA cleaning up columns for further purification. The recovered beads were resuspended in a solution of 300-600 mM imidazole, 50 mM sodium phosphate (pH 8.0), 300 mM NaCl and 0.01% Tween-20. Elution of the His-tagged T7 RNA polymerase from the beads was achieved by incubation for 5-30 min under orbital shaking at room temperature and subsequent magnetic isolation of the beads. Concentration and buffer exchange of the solution recovered with the eluted protein was achieved by centrifugation, for 10-25 min at 14000 g, with Amicon Ultra-0.5 3-10K centrifugal filter devices (Merck Millipore). A retentate containing the His-tagged T7 RNA polymerase was obtained after two washing cycles with 80 mM HEPES and 50 mM MgCl₂ 40 mM HEPES and 40 mM MgCl₂, or 1 M Tris-HCl, pH 8.0, 1 M NaCl and 100 mM EDTA (pH 8.0). Each washing step was followed by discard of the flow-through with the final retentate being reused in next transcription cycle. In each cycle, the resulting RNA samples were run on 2% agarose gels to monitor the reaction performance.

### 2.2. Results

An enzyme containing a polyhistidine-tag (His-tag) and with the ability of transcribing DNA into mRNA, the His-tagged T7 RNA polymerase, was used *in vitro* for in transcription and recycling purposes. Following transcription, and upon removal of the immobilized DNA template encoding for DHFR with the aid of a magnet, the His-tagged T7 RNA polymerase was isolated using affinity cobalt-coated magnetic beads. The produced RNA, recovered within the remaining supernatant, was purified and run on an agarose gel confirming the enzymatic performance. The retained enzyme was further eluted with imidazole and returned to a fresh *in vitro* transcription reaction cycle after concentration and buffer exchange. Figure 3 shows the successful reuse of the enzyme for at least 3 consecutive cycles of transcription (1 h) leading to the mRNA product of interest. The resulting RNA could be then transferred to a separate translation reaction unit for further protein synthesis.

### Example 3: Production of a small peptide and a functional protein by in vitro translation fed with mRNA previously synthetized in a separate in vitro transcription reaction

### 3.1. In vitro translation

A customized PURExpress kit depleted from the His-tagged T7 RNA polymerase (ΔT7 PURE), supplied by NEB, was used as described by the manufacturer for *in vitro* translation (IVTL) of previously synthetized mRNA according to Example 1. In brief, 0.75 µL of Ribolock RNase inhibitor (40 U/µL) from Thermo Fisher Scientific was added to a reaction mixture consisting of two commercial solutions from the ΔT7 PURE kit, A (10 µL) and B (7.5 µL). The RNA, previously produced by in *vitro* transcription, was transferred to the in *vitro* translation reaction with a maximum input volume of 11.75 µL per reaction, and incubated in a final volume up to 30 µL for 3 h at 37 °C. Transcription reaction volumes up to 500 µL enabled the preparation of aliquots containing 1-6 µg or 2.5-25 µg RNA codifying for a peptide (M(GGGGS)₄) and a protein (DHFR), respectively. The RNA samples were translated *in vitro* using low-binding or standard Eppendorf tubes, without or under orbital shaking at 200 rpm. Upon completion of the reaction, the samples were cooled down on ice and the product was stored at < -70 °C before further characterization by High Resolution Mass Spectroscopy (HRMS) or activity assessment.

### 3.2, M(GGGGS)₄ peptide identification by Ultra Performance Liquid Chromatography Coupled to a High Resolution Mass Spectrometer (UPLC-HRMS)

Detection of the M(GGGGS)₄ peptide within samples prepared by uncoupled *in vitro* transcription and translation was performed by liquid chromatography-mass spectrometry (LC-MS). The system consisted of an Acquity H-Class Bio ultrahigh pressure liquid chromatography (UPLC) system (Waters, Milford, United States of Amerika) configured with a binary solvent manager, a flow-through needle autosampler and a column manager. The UPLC was coupled on-line to a quadrupole - ion mobility spectrometer - time-of-flight mass spectrometer (Vion IMS-QToF MS, Waters). The system was controlled by Waters Connect 200831 with Unifi 1.9.13.9. Aliquots of 2 µL sample were loaded onto an analytical column (Acquity HSS T3, 2.1 mm ID × 100 mm, 1.8 µm particle and 100 Å pore size, Waters) set at 40 °C with a column flow rate of 0.6 mL/min. Samples were eluted with the following binary gradient: starting with 0% eluent B, the gradient was linearly ramped to 10% eluent B in 3 min, then to 5% B in 1 min and held at this composition for 1 min, where eluent A consisted of 0.1% formic acid (Biosolve, Valkenswaard, The Netherlands) in water (Milli-Q, Millipore), and eluent B consisted of 0.1% formic acid in acetonitrile (Biosolve, Valkenswaard, The Netherlands). The column was re-equilibrated for 1 min prior to each analysis. The mass spectrometry system was equipped with a Lockspray source operated in electrospray ionization (ESI) mode. A spray voltage of 0.8 kV was applied in positive ionization mode and the desolvation temperature was set to 550 °C, with a desolvation gas flow of 1000 L/h and cone gas flow of 50 L/h. The source temperature was set to 120 °C. The sample cone voltage was set to 30 V and the source offset to 80 V. Prior to analysis of the study samples and reference standards, an automated set-up and mass calibration was performed in negative ionization mode. Further, a multiple reaction monitoring mode with one transition and M(GGGGS)*₄* as the precursor/product (m/z 705.77) was used, together with the collision energy set for nitrogen at 6 V and dwell time set to 100 ms.

Study samples obtained using 1-6 µg of RNA as input from previous transcription into the translation reaction, were diluted in sterile MilliQ water prior to injection onto the UPLC. As a positive control, the reference peptide (GenScript) was diluted with sterile MilliQ water to a concentration of 10 ng/mL. Moreover, the PURE system without mRNA input was used as a negative control. The resulting chromatograms were extracted with a 10 ppm window around the target mass.

### 3.3. Dihydrofolate reductase protein activity assessment

The activity of the protein produced by uncoupled *in vitro* transcription and translation was assessed using a colorimetric dihydrofolate reductase (DHFR) assay kit (Abcam) for analysis of 1 µL samples in 96-well plates. According to the manufacturer, absorbance of the samples directly obtained from the PURE system and standard references was measured at 340 nm in a microplate reader, with the generated curves being background corrected for a blank. The ΔT7 PURE system incubated in the absence of an mRNA input was used as the negative control. The study samples were preferably diluted 5-fold in MilliQ water prior to the enzymatic analysis. Commercial DHFR (R&D systems) was used for the preparation of standard reference solutions with known protein concentrations (2.5-200 ng/µL), which were used as the positive controls, for comparison and calibration purposes.

### 3.4. Results

A flexible GS linker (M(GGGGS)₄) for heterologous expression and dihydrofolate reductase (DHFR) were produced, as a test peptide and an enzymatically active protein, by an uncoupled *in vitro* transcription and translation set-up. The spatial separation between transcription and translation reactions with an intermediate step of RNA purification allows controlling the RNA input (e.g., isolation of RNA from the DNA and T7 RNA polymerase, with adjustment of RNA concentration in the transferred solution) to the translation reaction. Upon elution, mRNA aliquots were transferred to a commercial PURE Express system depleted from the T7 RNA polymerase, the ΔT7 PURE system. Thus, the enzymatic machinery from the commercial ΔT7 PURE kit could only be used for *in vitro* translation purposes. Moreover, the DNAse treatment, performed before transferring mRNA into the translation compartment, guarantees that the observed peptide or protein yields can only be directly related to the mRNA produced in the transcription compartment. The DNAse treatment, was solely employed confirmation purposes in this example.

The production of a test peptide M(GGGGS)₄, with an absolute mass of m/z 705.77, was confirmed by UPLC-HRMS analysis, as shown in the chromatograms presented in Figure 4. The compound identification was further supported by the detection of the 10 ng/mL reference (Figure 4B) and the background level confirmed in the negative control (Figure 4A) at the corresponding mass and expected retention time for the peptide. Moreover, Figure 4 reveals a proportional increase in MS intensity in related to the RNA input used for *in vitro* translation of 1 µg (P1, Figure 4C) and 6 µg (P2, Figure 4D) of RNA samples.

Finally, the formation of the DHFR protein was supported by SDS-PAGE of the product directly obtained after translation, with the appearance of a 16 kDa band in the gel depicted in Figure 5A. The absence of the DHFR band in the negative control corroborates the specificity of the assay. Moreover, the protein was proven to be enzymatically active by using a DHFR activity assay kit. The test principle is based on the ability of DHFR to catalyse the reduction of 7,8-dihydrofolate (DHF) to 5,6,7,8-tetrahydrofolate (THF), using β-Nicotinamide adenine dinucleotide phosphate reduced tetrasodium salt (NADPH) as a cofactor. The progress of the reaction was monitored by the decrease in absorbance at 340 nm, related to the oxidation of NADPH to NADP⁺. On one hand, Figure 5B illustrates the plateau reached in the negative control, in absence of the enzyme. On the other hand, a steady decrease in absorbance is observed for the 5-fold diluted sample, directly obtained after 3 h of IVTL fed with 25 µg RNA, before the plateau. The decrease in absorbance of the study sample is positioned between the corresponding curves obtained for the reference standard solutions, or positive controls, with 100 and 200 ng/µL DHFR. These results confirm both the formation and activity of the DHFR protein synthetized with the uncoupled *in vitro* transcription and translation method as described in this invention.

## Claims

1. A method for *in vitro* synthesis of a peptide or protein comprising:
a) transcribing a DNA into RNA in a first reaction container comprising the DNA and an enzyme capable of transcribing DNA into RNA,
b) introducing the RNA into a second reaction container, and
c) translating the RNA into a peptide or protein in the second reaction container,
wherein the first reaction container and the second reaction container are connected.

2. The method according to claim 1, wherein the method is performed using a reconstituted cell-free protein synthesis system.

3. The method of claim 2, wherein the components constituting the reconstituted cell-free protein synthesis system are purified components.

4. The method according to any one of the preceding claims, wherein the first reaction container comprises DNA immobilized on a solid support.

5. The method according to any one of the preceding claims, wherein the method is performed in a system according to claim 12.

6. The method according to any one of the preceding claims, further comprising separating the DNA and the RNA prior to introducing the RNA into the second reaction container.

7. The method according to any one of the preceding claims, further comprising separating the enzyme capable of transcribing DNA into RNA and the RNA prior to introducing the RNA into the second reaction container, preferably wherein said separating of the enzyme and the RNA is performed in a separation module.

8. The method according to claim 7, wherein the enzyme capable of transcribing DNA into RNA that is separated from the RNA is reintroduced into the first reaction container.

9. The method according to any one of the preceding claims wherein the method is performed in a continuous or semi-continuous manner, whereby the RNA is continuously or semi-continuously introduced into the second reaction container and/or the enzyme capable of transcribing DNA into RNA that is separated from the RNA is continuously or semi-continuously reintroduced into the first reaction container.

10. The method according to any one of the preceding claims, wherein the first reaction container further comprises a reaction solution comprising a buffer and ribonucleotides, preferably nucleoside triphosphates (NTPs).

11. The method according to any one of the preceding claims, wherein the second reaction container comprises ribosomes, tRNAs, amino acids, aminoacylated tRNAs, catalysts for tRNA aminoacylation and/or translation factors for protein synthesis.

12. A system for reconstituted cell-free protein synthesis, comprising:
- a first reaction container comprising a reaction solution comprising an enzyme capable of transcribing DNA into RNA and ribonucleotides, preferably nuceloside triphosphates (NTPs),
- a second reaction container comprising a reaction solution comprising ribosomes, tRNAs, amino acids, aminoacylated tRNAs, catalysts for tRNAs aminoacylation and/or translation factors for protein synthesis, and
- a conduit for conducting RNA from the first reaction container into the second reaction container.

13. The system according to claim 12, wherein the components constituting the reconstituted cell-free protein synthesis system are purified components.

14. The system according to claim 12 or 13 further comprising a support for immobilization of DNA.

15. The method or system according to any one of the preceding claims, wherein the first reaction container comprises an access for insertion of a solid support comprising DNA immobilized thereto into the first reaction container such that the immobilized DNA is in contact with the reaction solution comprising the enzyme capable of transcribing DNA into RNA and the ribonucleotides.

16. The method or system according to any one of the preceding claims, further comprising a separation module for separating the enzyme capable of transcribing DNA into RNA and RNA, which interrupts the conduit for conducting RNA from the first reaction container to the second reaction container.
